# EUROPEAN PATENT APPLICATION

(11) **EP 2 939 702 A1**
(43) Date of publication of application: **04.11.2015**
(21) Application number: 13868381.8
(22) Date of filing: 20.12.2013
(51) Int. Cl.: A61M 25/10

(54) **CATHETER BALLOON AND CATHETER**

(30) Priority: 25.12.2012 JP 2012281705
(71) Applicant: Tokai Medical Products, Inc., Aichi 486-0808 (JP)
(72) Inventor: TSUTSUI, Nobumasa, Kasugai-shi Aichi 486-0808 (JP); TSUTSUI, Yasuhiro, Kasugai-shi Aichi 486-0808 (JP); MURAKI, Yasuhiro, Kasugai-shi Aichi 486-0808 (JP)
(74) Representative: Zeuner Summerer Stütz
(86) International application number: PCT/JP2013/084197
(87) International publication number: WO 2014/103908

(57) **Abstract**

[Object] Provided is an expansible catheter balloon that has a spreadable outer diameter controllable easily in treatments for a calcific aortic stenosis, rheumatic and congenital aortic stenoses and others, and that can be effectively prevented from being freely shifted and can easily be fixed at a predetermined position.

[Solution] The catheter balloon 10 includes a balloon 20 having shoulder parts 23 made of a non-expansible material or made low-expansible and an expandable waist part 30, and a catheter having a catheter body 70 to which this balloon 20 is fitted.

## Description

### [Technical Field]

The present invention relates to a catheter balloon, and a catheter.

### [Background Art]

Causes for a stenotic aortic lesion are, for example, an acquired calcific or rheumatic aortic stenosis, and a congenital aortic stenosis. For example, about the calcific aortic stenosis, at the time when the lesion thereof is deteriorated, the calcification of the valve cusp advances so that the valve cusp gradually hardens. As a result, the mobile scope thereof is gradually restricted to cause cardiac insufficiency. For this reason, in the case of a severe valve cusp stenosis, it is necessary to treat the stenosis by operation.

In order to treat such a disease, balloon valvuloplasty is suggested as a low-invasive treating method for spreading a valve undergoing a stenosis, such as a calcific aortic stenosis, or a rheumatic or congenital aortic stenosis. An ordinary operation for the present treating method about the calcific aortic stenosis is as follows: first, a catheter having a deflated balloon is percutaneously inserted into a vein or artery, and then the balloon is arranged inside a cardiac valve required to be treated. Next, by spreading the balloon, the valve cusp is spread to pulverize a calcific portion depositing on the valve cusp. In this way, the softness of the valve cusp is restored to improve the mobile scope. After it is confirmed that the ejection quantity of the blood flow increases, the balloon is deflated and the catheter is removed from the inside of the body.

In order to spread the valve cusp of the aorta largely, the catheter balloon used in the present treatment may be made of a highly elastic material such as latex or silicone to cause the whole of the balloon to have expansibility. By using the highly elastic material to make the balloon expansible, advantages are produced that the initial outer diameter thereof can be made small and further the flexibility thereof facilitates the invasion and advance of the balloon into the blood vessel. Furthermore, the balloon has an advantage that when the balloon is removed from the blood vessel, the balloon can easily be withdrawn since the outer diameter of the balloon is made small after the deflation. However, the balloon made of the highly elastic material such as latex or silicone can be arbitrarily made large in diameter to cause a problem of requiring an instrument or means for precisely controlling the spread outer diameter of the expansible balloon.

About any existing balloon, the shape thereof is cylindrical or ovoid, and further the balloon is easily slipped in blood or on a vascular wall. Thus, the balloon is freely shifted, and there remains a problem that a considerable skill and attentiveness are required to locate the balloon at a predetermined position.

### [Citation List]

### [Patent Document]

Patent Document 1: JP 2005-537856 A

### [Summary of Invention]

### [Problem to be Solved]

Thus, the present invention has been made in light of the above-mentioned problems, and an object thereof is to provide a catheter balloon, and a catheter, the balloon being an expansible balloon that has a spreadable outer diameter controllable easily in treatments for a calcific aortic stenosis, rheumatic and congenital aortic stenoses and others, and that can be effectively prevented from being freely shifted and can easily be fixed at a predetermined position.

### [Solution to Problem]

In order to attain the object, the present invention is as follows:

The catheter balloon of the present invention includes a balloon including:
non-expansive or low-expansive cylindrical shoulder parts formed at both end sides, respectively; and
a waist part which is formed between the shoulder parts, is lower in extension ratio than the shoulder parts when the individual parts are shaped, is shorter in diameter than the shoulder parts, and is increasable in diameter by spreading the balloon.

According to the present invention, the shoulder parts of the balloon are made non-expansive or low-expansive; thus, even when the internal pressure thereof is made high, the balloon can be prevented from continuing to be inflated to be excessively largely increased in diameter as seen in an expansive balloon. Consequently, the balloon can be prevented from being excessively spread to damage tissues therearound. At a pressure not more than pressures permitting the waist part to be expanded, the shoulder parts at both sides of the balloon, and the waist part are together spread to a predetermined diameter; however, these parts are not expanded. Thus, about the catheter balloon, the waist part is formed into a concave form. This manner makes it possible to arrange an annulus naturally at the waist part to sandwich the annulus between the shoulder parts.

Furthermore, it is allowable that the catheter balloon according to the present invention is in the form of a substantial gourd when a pressure less than a predetermined pressure is applied thereinto, and the waist part is mainly expanded when the predetermined pressure or more is applied, so that the diameter of the waist part becomes equivalent to or slightly smaller than that of the shoulder parts. This structure makes it possible to spread a valve cusp by an inflation of the waist part, so as to pulverize a calcified portion of the valve cusp.

Additionally, it is allowable in the catheter balloon according to the present invention that the shoulder parts are made of a plastic material extended sufficiently when the material is shaped, the waist part is made of a plastic material extended insufficiently when the material is shaped, and the waist part is further extended when the balloon is used to spread the waist part, whereby the diameter thereof is spread up to a diameter equivalent to that of the shoulder parts. Since the shoulder parts are extended closely to the limit of the extension ratio of the raw material, this structure makes it possible to prevent the shoulder parts from continuing to be inflated as seen in an expansible balloon to become too large in diameter even when the internal pressure thereof is increased as far as the increased pressure is not more than the endurable pressure of the parts. Moreover, this structure makes it possible to prevent the waist part from being expanded over the diameter of the shoulder parts. This structure makes it possible to prevent tissues around the balloon surely from being broken by an excessive spread of the balloon. The structure also makes it possible to produce, easily through a single step, a catheter having expansivity at only its waist part.

Furthermore, it is allowable in the catheter balloon according to the present invention that only the shoulder parts are spread at a pressure less than a predetermined pressure, so that the balloon is made into a substantial gourd form; and the diameter of the waist part is equal to or less than that of the shoulder parts even when a pressure of 3.0 atm or more is applied to the balloon. According to this structure, the waist part is caught by an annulus when a calcified portion of the valve cusp is pulverized. Thus, the balloon can be prevented from being freely shifted.

Furthermore, the catheter of the present invention includes: an outer tube; and the above-mentioned catheter balloon that is connected to a lumen of the outer tube which is a lumen for injecting a spreading fluid.

### [Advantageous Effects of Invention]

According to the catheter balloon, and the catheter according to the present invention, the spreadable outer diameter of the balloon, which is expandable, can easily be controlled and further the balloon can be effectively prevented from being freely shifted in treatments for calcific, rheumatic and congenital aortic stenosis, and others. Moreover, the present invention can provide a catheter balloon and a method for producing a catheter, this balloon and a balloon of this catheter being capable of being easily fixed at a predetermined position.

### [Brief Description of Drawings]

Fig. 1A is a schematic view illustrating a state of a catheter balloon 10 according to an embodiment that shoulder parts thereof are spread; and Fig. 1B is a schematic view illustrating a state of a balloon 20 according to the embodiment that a waist part 30 thereof is expanded.
Fig. 2 is a view illustrating a state of the catheter balloon 10 according to the embodiment that the waist part thereof is expanded.
Figs. 3(A) to 3(C) are views illustrating a process of a method using the catheter balloon 10 according to the embodiment.
Fig. 4 is a graph showing a change in the internal pressure of a balloon, and a change in the outer diameter of the catheter balloon.

### [Embodiment Carrying Out the Invention]

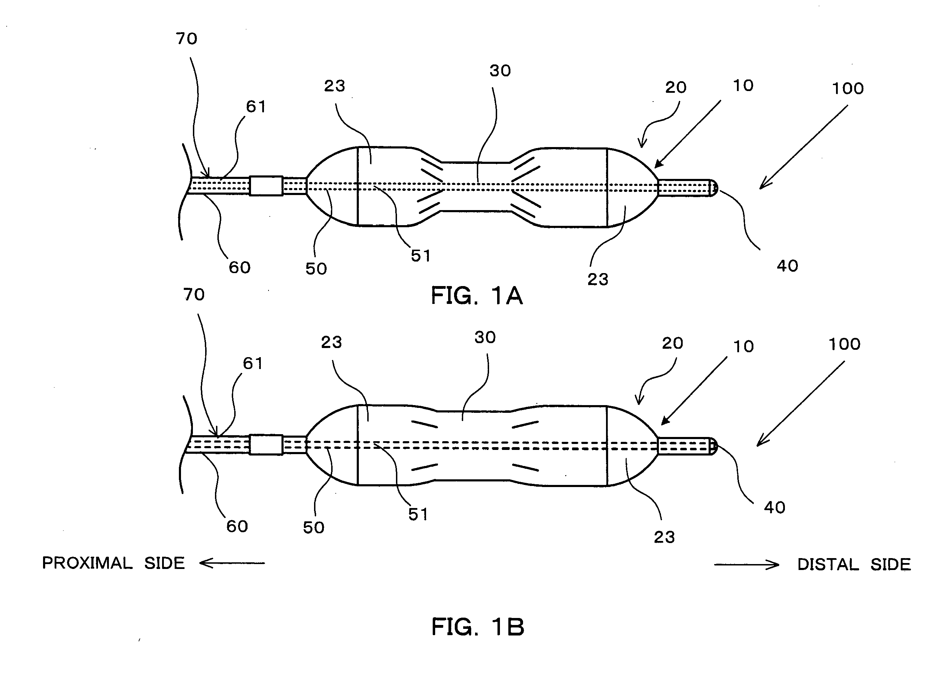

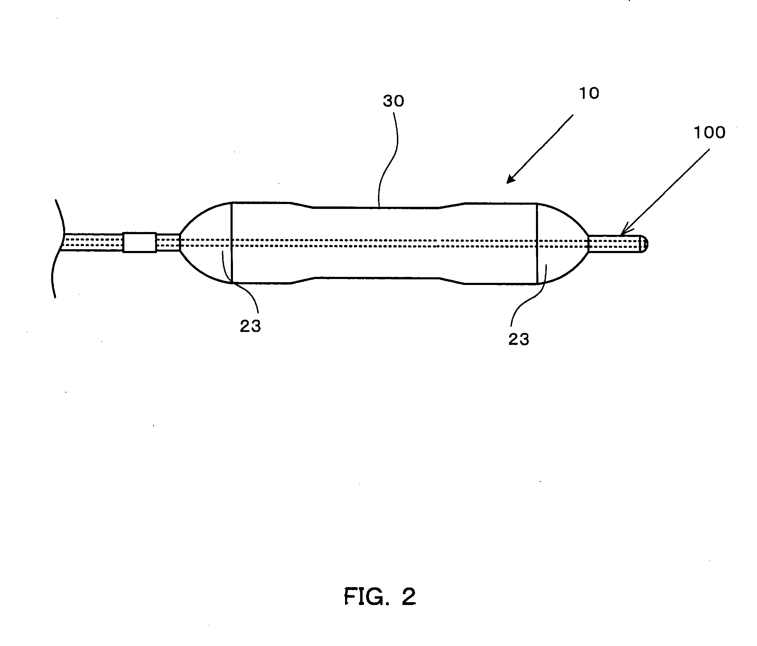

Hereinafter, a detailed description will be made along the drawings about a catheter balloon 10, a catheter 100, and a method for producing the catheter balloon 10 according to an embodiment. Fig. 1A is a schematic view illustrating a state of the catheter balloon 10 according to the embodiment that only shoulder parts 23 thereof are spread. Fig. 1B is a schematic view illustrating a state thereof that a waist part 30 thereof is expanded. Fig. 2 is a schematic view illustrating a state thereof that the waist part 30 of the catheter balloon 10 according to the embodiment is substantially expanded. In the present specification, the word "spread" means that the balloon is developed merely by an increase in the internal pressure thereof but does not mean that the balloon itself is substantially expanded to be inflated. The word "expand" means that the balloon itself is expanded to be made longer in diameter. The word "extend" means that a heated plastic film is stretched to align molecules thereof.

As illustrated in Fig. 1, the catheter balloon 10 according to the present embodiment has the balloon 20, which has the shoulder parts 23 made of a non-expansive material or made into a low-expansivity and the waist part 30 having expansivity, and a catheter body 70 to which this balloon 20 is fitted.

The shoulder parts 23 are made non-expansive or low-expansive. When pressured, the shoulder parts 23 are spread. The spread of the shoulder parts 20 by an initial pressurization thereto means that their cylindrical portion is merely spread-opened, and the raw material itself of the parts is not expanded. Thus, the shoulder parts 23 are spread even by a relatively low pressure of, for example, 0.05 atm or less. Thereafter, even when the pressure is increased, the shoulder parts are hardly expanded to give an expansion ratio of about 20% or less since the shoulder parts are made into low-expansive or non-expansive.

The waist part 30 is positioned in a middle position of the balloon 20, and has expansivity. The waist part 30 has a diameter shorter than the spreadable diameter of the shoulder parts 23 in the state that no force for expansion is applied thereto. For this reason, about the catheter balloon 10, the waist part 30 is intentionally kept narrow until the pressure applied thereto reaches a predetermined pressure, so that the balloon 10 is made in a gourd form as a whole. When the pressure is further increased, the waist part 30 can be expanded and inflated.

The material used for the balloon 20 may be an already-known material that may be of various types. Examples thereof include nylon, polyvinyl chloride, polyethylene, polyurethane, polyether amide block copolymer, polyethylene terephthalate, olefin based polymers such as polypropylene, and a copolymer made of any combination of two or more of these polymers. The balloon 20 may not be necessarily made of a single material. The balloon 20 may be a bi-layered balloon composed of an inner layer and an outer layer that are different from each other in raw material. Such a bi-layered balloon can be produced by shaping a tube formed by bi-layer extrusion. By adopting the expansion ratio in the above-mentioned range, the balloon can be prevented from being excessively spread. The balloon 20 is formed into a length of 30 mm to 80 mm, preferably 40 to 70 mm. The maximum spreadable diameter thereof is preferably from 10 mm to 25 mm. Of course, the maximum spreadable diameter is not limited into this range. In accordance with the maximum spreadable diameter, the catheter is usable for arteries, veins, bodily cavities, and other hollow organs of the human body.

In an example of a method for producing the balloon 20, a plastic tube is formed by extrusion, and this tube is heated and then extended and shaped to spread only the shoulder parts 23. In this way, the balloon 20 can be produced. By drawing only the shoulder parts 23, molecules constituting the shoulder parts 23 of the balloon are oriented. Thus, the shoulder parts 23 come to be lowered in expansivity, or not to have expansivity. In the meantime, the waist part 30 is insufficiently extended to be made larger in thickness than the shoulder parts. Moreover, by increasing the internal pressure, the waist part 30 is expanded while extended. As described herein, the shoulder parts 23 of the single tube are extended to result in a difference in expansion ratio. In this way, the balloon is produced. As, in the production, the waist part 30 is being expanded, the expansion ratio of the waist part 30 is approaching the extension ratio of the shoulder parts 23 so that the former ratio is approaching a value equal to the ratio of the shoulder parts 23. When the waist part 30 is extended to the same size as the shoulder parts 23, the waist and shoulder parts theoretically have the same expansion ratio. The sufficiently extended plastic material denotes that the plastic material has an extension ratio of 90% to 100%. More preferably, the shoulder parts 23 have an extension ratio of 95% to 100%, and should be advisably extended into a draw ratio close to about 100% as much as possible. The insufficiently extended plastic material denotes that the extension ratio thereof is from about 60% to 80%. More preferably, the waist part 30 has an extension ratio of about 60% to 70%. After the waist part is spread to have an extension ratio close to that of the shoulder parts, the balloon is never spread over the spread limit of the whole. Thus, a possibility can be decreased that the balloon is excessively spread to damage tissues around the balloon.

The catheter balloon 10 is arranged at the distal end of the catheter body 70 to constitute the catheter 100. Specifically, as illustrated in Fig. 1A, the catheter 100 has a tip part 40 fitted to the tip of the distal side of the catheter body 70, an inner tube 50 having a guide wire lumen 51 connected to this tip part 40, the balloon 20 arranged around this guide wire lumen 51, and an outer tube 60 having a spreading-fluid-injecting lumen 61 connected to the balloon lumen 52 of this balloon 20. The balloon 20 is inflated by a spreading fluid passing through this spreading-fluid-injecting lumen 61.

About the catheter balloon 10 produced as described above, the waist part 30 keeps the diameter of the middle of the balloon 20 small. Thus, when the spreading fluid is inserted into the balloon 20 at a predetermined pressure or less, specifically at a pressure permitting the waist part 30 to be spread or inflated, or lower, only the shoulder parts 23 are being spread while the waist part 30 of the balloon 20 keeps the initial diameter thereof. Subsequently, the spread of the shoulder parts 23 and the other reaches into an equilibrium. The spread of the shoulder parts 23 causes the balloon 20, which has been folded up, to merely open, and thus the raw material itself of the balloon 20 is not expanded. Accordingly, the shoulder parts 23 are spread even at a relatively low pressure, for example, 0.05 atm or less. However, about the waist part 30, the raw material itself thereof needs to be expanded. Thus, at a low pressure, the waist part 30 is hardly expanded so that the waist part 30 is kept to have the pre-expansion diameter. Consequently, as illustrated in Fig. 1A, the balloon 20 is made, as a whole, in the so-called gourd form, which is such a form that the waist part 30 of the center of the balloon 20 is narrow and the shoulder parts 23 are thick. When a valve cusp or annulus is fitted onto this waist part 30, the balloon 20 is easily arranged at a predetermined position so that the balloon 20 can be prevented from being freely shifted. By changing this state further to a state that the spreading fluid is injected into the balloon 20 to increase the internal pressure of the balloon 20, the waist part 30 is expanded to be increased in diameter. At this time, the shoulder parts 23 of the balloon 20 are non-expansive or low-expansive, and thus after the equilibrium state, the shoulder parts 23 are not spread or are only slightly spread. Furthermore, when the spreading fluid is further injected into the balloon 20 to increase its internal pressure, the waist part 30 is expanded. Thus, as illustrated in Fig. 1B, the diameter of the waist part 30 is being spread. When the spreading fluid is further injected thereinto, the waist part 30 of the balloon 20 is spread, as illustrated in Fig. 2A, into a diameter equivalent to that of the shoulder parts 23. However, the balloon 20 is merely spread slightly more largely than this diameter since the balloon 20 is non-expansive or low-expansive. It is therefore possible to prevent the diameter from continuing to be expanded as seen in any expansive balloon, so that tissues around the balloon are prevented from being damaged. As described above, it does not happen that the diameter of the waist part 30 is excessively increased over that of the shoulder parts 23.

The following will describe how to use the above-mentioned catheter for valvuloplasty. According to the Seldinger technique, the catheter 100 is introduced through a thigh or upper arm artery, or left subclavian artery. Alternatively, after a surgical incision, the catheter 100 is directly inserted into an aortic valve, so that the catheter 100 can be anterogradely inserted. Hereinafter, as an example, a case will be described where at the time of a detention of the catheter balloon 10 in a transcatheter aortic valve replacement, the balloon 10 is detained in a valve cusp by an approach through a thigh. It is however needless to say that various detaining techniques are usable in accordance with the present invention.

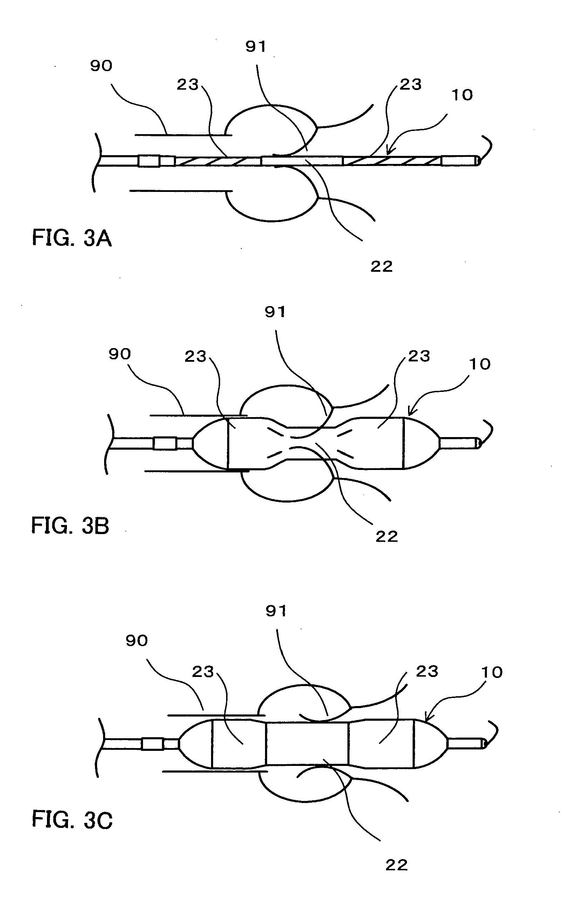

A puncture needle is stuck into a femoral artery (or an iliac artery inside the pelvis), which is near person's groin. Thereafter, a guide wire is inserted into the blood vessel. While the guide wire is being shifted along the blood vessel, the guide wire is being slipped to be inserted into the blood vessel. The guide wire is passed through the aortic valve, and then indwelled. Thereafter, while the position of the guide wire is being monitored on a roentgenoscopic screen, the catheter balloon 10 is retrogradely advanced to a target position. Next, as illustrated in Fig. 3(A), the catheter balloon 10 is detained to position the shoulder part 23 at the distal end side of the balloon 10 inside the left ventricle outflow tract, and position the waist part 30 at a valve cusp 91 of an aorta 90.

As illustrated in Fig. 3(B), a spreading fluid is introduced into the catheter balloon 10 to increase the pressure therein up to about 0.1 atm to 0.5 atm (the pressure in the balloon 20 is made slightly higher than that outside the balloon). In this way, the balloon 20 is spread. According to this pressure, the shoulder part 23 at the proximal side of the catheter and the shoulder part 23 at the distal side are spread while the waist part 30 is not spread to keep the diameter thereof small. In this state, about the catheter balloon 10, the spreadable diameter of the shoulder parts 23 is selected into a size suitable for the valve cusp and the LVOT. For this reason, the annulus is arranged to be sandwiched between the shoulder parts 23 at both the sides, so that the waist part 30 is naturally arranged at the valve cusp. Consequently, the catheter balloon 10 can be arranged at a desired position to be prevented from being freely shifted.

Next, the pressure inside the balloon 20 is increased, and the spreading fluid is introduced thereinto to give a pressure in the range of 0.5 atm to 5.0 atm, preferably 1.0 atm to 3.0 atm. As a result, the waist part 30 is spread to start pushing and moving the valve cusp outward. The spread of the waist part 30 pushes and moves the valve cusp to be opened, so that its calcified portion is pulverized.

As the pressure inside the balloon 20 is further increased, the diameter of the waist part 30 is gradually increased. Thus, as illustrated in Fig. 3(C), the waist part 30 is spread into an outer diameter equivalent to that of the shoulder parts 23. The outer diameter of the waist part 30 is not spread further, thereby completely preventing a risk that the balloon is excessively spread to damage tissues around the balloon. The balloon 20 is deflated in a short period since the blockage of the blood flow is restricted into a short period. When the balloon is spread, a contrast medium may be injected into the balloon 20. In this way, it is possible to roentgenize the valve cusp in the state that the valve cusp is spread. Thus, the diameter of the spread valve cusp can be estimated.

### (Example)

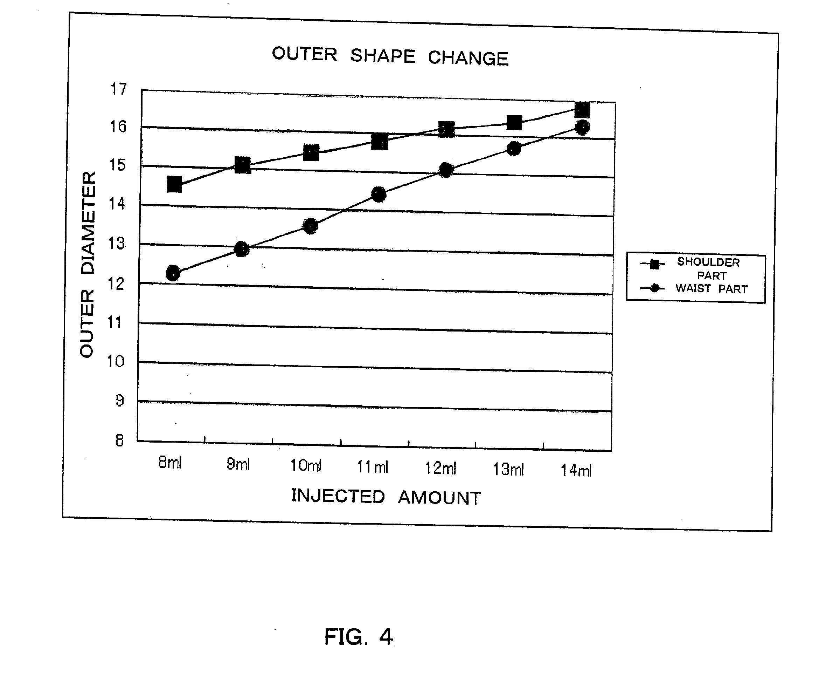

Hereinafter, experimental results are shown about the pressure and the outer diameter in a working example. The present example merely shows one aspect of the invention. Thus, raw material and forms usable in the present invention are not limited to the present raw materials and form. In a balloon 20 having a waist part 30 of 12 mm in diameter, shoulder parts 23 of 15 mm in diameter, and a length of 40 mm, a spreading fluid was injected thereinto in a volume over a predetermined volume (8 mL) permitting the waist part to start spreading. At the time, measurements were made about a change in the outer diameter of the waist part 30 of the balloon 10, and a change in the outer diameter of the shoulder parts 23. In a method for the measurement, the measurement of the outer diameter of each of the parts was started when the fluid was injected into the balloon 10 in a volume over 8 mL after the start of the injection. Thereafter, the fluid was injected thereinto in a constant volume per time, and the outer diameter of each of the parts was measured. The measurement results in this case are as follows: When the injected volume was 8 mL, the outer diameter of the waist part was 12.3 mm and that of the shoulder parts 14.6 mm. When the injected volume was 9 mL, the outer diameter of the waist part was 13.0 mm and that of the shoulder parts 15.1 mm. When the injected volume was 10 mL, the outer diameter of the waist part was 13.6 mm and that of the shoulder parts 15.5 mm. When the injected volume was 11 mL, the outer diameter of the waist part was 14.5 mm and that of the shoulder parts 15.8 mm. When the injected volume was 12 mL, the outer diameter of the waist part was 15.1 mm and that of the shoulder parts 16.2 mm. When the injected volume was 13 mL, the outer diameter of the waist part was 15.7 mm and that of the shoulder parts 16.4 mm. When the injected volume was 14 mL, the outer diameter of the waist part was 16.3 mm and that of the shoulder parts 16.8 mm. A graph of the measurement results is demonstrated in Fig. 4.

According to the measurement results, as the injected amount is increased, the outer diameter of the waist part 30 is increased while the increase rate of the outer diameter of the shoulder parts 23 is lower than that of the waist part 30. The expansion ratio of the outer diameter of the shoulder parts is held in the range of 10% or less. Even when the injection is continued up to the limit thereof, the waist part 30 and the shoulder parts 23 do not have the same outer diameter. The diameter of the waist part is slightly shorter so that a convex-concave form is produced. Thus, the balloon can be prevented from being slipped to be freely shifted.

The present invention is never limited to the above-mentioned embodiments. Of course, the present invention can be carried out in various forms as far as the forms fall under the technical scope of the present invention.

First, the catheter balloon 10 and the catheter 100 according to the present embodiment have been described through examples thereof in transcatheter aortic valve replacement. However, they are usable for a valvuloplasty of a stenotic aortic valve or pulmonary valve or for dilating all stenotic constrictions in blood vessels. They are also usable when an aortic valve cusp is pre-spread before a percutaneous aortic valve or any other artificial device used for aortic valve repair, replacement or transplantation is detained. Examples of the blood vessel to which the invention is applicable include all blood vessels of the body, such as coronary arteries, peripheral arteries, veins, the gullet, the trachea, enteric blood vessels, the bile duct, and the ureter of the body.

### [Industrial Applicability]

As has been demonstrated in the above-mentioned embodiment, the present invention is usable for intervention operations, in particular, for preventing a blood clot or debris from being scattered.

### [Reference Signs List]

10: catheter balloon, 20: balloon, 22: waist part, 23: shoulder part, 30: waist part, 40: tip part, 50: inner tube, 51: guide wire lumen, 52: balloon lumen, 60: outer tube, 61: spreading-fluid-injecting lumen, 70: catheter body, 90: aorta, 91: valve cusp, and 100: catheter

## Claims

1. A catheter balloon, comprising a balloon comprising:
non-expansive or low-expansive cylindrical shoulder parts formed at both end sides, respectively; and
a waist part which is formed between the shoulder parts, is lower in extension ratio than the shoulder parts when the individual parts are shaped, is shorter in diameter than the shoulder parts, and is increasable in diameter by spreading the balloon.

2. The catheter balloon according to claim 1, wherein:
the balloon is in the form of a substantial gourd when a pressure less than a predetermined pressure is applied thereinto; and
the waist part is mainly expanded when the predetermined pressure or more is applied, so that the diameter of the waist part becomes equivalent to or slightly smaller than that of the shoulder parts.

3. The catheter balloon according to claim 1 or 2, wherein:
the shoulder parts are made of a plastic material extended sufficiently when the material is shaped,
the waist part is made of a plastic material extended insufficiently when the material is shaped, and
the waist part is further extended when the balloon is used to spread the waist part, whereby the diameter thereof is spread up to a diameter equivalent to that of the shoulder parts.

4. A catheter, comprising:
an outer tube, and
the catheter balloon according to any one of claims 1 to 3, wherein the balloon is connected to a lumen of the outer tube which is a lumen for injecting a spreading fluid.
